# EUROPEAN PATENT APPLICATION

(11) **EP 1 935 982 A2**
(43) Date of publication of application: **25.06.2008**
(21) Application number: 06819976.9
(22) Date of filing: 07.09.2006
(51) Int. Cl.: C12Q 1/02, C07K 14/47, C12R 1/91, C12Q 1/68

(54) **DIAGNOSTIC KIT AND METHOD FOR THE QUANTITATIVE DETERMINATION OF THE EXPRESSION OF THE MAXI-K CHANNEL**

(30) Priority: 08.09.2005 ES 200502247
(71) Applicant: Universidad de Sevilla, E-41012 Sevilla (ES)
(72) Inventor: NAVARRO ANTOLÍN, Francisco Javier, E-41009 Sevilla (ES); LEVITSKY, Konstantin, E-41009 Sevilla (ES); LÓPEZ BARNEO, José, E-41009 Sevilla (ES); CALDERÓN SÁNCHEZ, Eva, E-41009 Sevilla (ES); ORDÓÑEZ FERNÁNDEZ, Antonio, E-41009 Sevilla (ES); VILLAR LÓPEZ, José, E-41009 Sevilla (ES); CARMONA BERNAL, Carmen, E-41009 Sevilla (ES); CAPOTE GIL, Francisco, E-41009 Sevilla (ES)
(74) Representative: Elzaburu Marquez, Alberto
(86) International application number: PCT/ES2006/000504
(87) International publication number: WO 2007/028845

(57) **Abstract**

The invention relates to a diagnostic kit and method for the quantitative determination of the expression of the KCNM genes that are involved in human arterial hypertension in peripheral blood leukocytes from a whole blood sample and, more specifically, of the expression of the levels of mRNA and/or proteins from the alpha and beta1 subunits of the maxi-K ion channel, i.e. genes KCNMA1 and KCNMB1

SEQUENCE LISTING

<110>  Universidad de Sevilla

<120>  Diagnostic kit and Method for the Quantitative Determination of
 the Expression of the Maxi-K Channel

<130>  ES-UNIV.SEV.

<160>  6

<170>  PatentIn version 3.1


<210>  1
<211>  20
<212>  DNA
<213>  Homo sapiens

<400>  1
tcagcagatg catgcctgat                                                    20


<210>  2
<211>  23
<212>  DNA
<213>  Homo sapiens

<400>  2
tctcatgata ttcgaggcat cct                                                23


<210>  3
<211>  24
<212>  DNA
<213>  Homo sapiens

<400>  3
gtgtgccgtc atcacctact acat                                               24


<210>  4
<211>  18
<212>  DNA
<213>  Homo sapiens

<400>  4
cctgggtcca cacgcttt                                                      18


<210>  5
<211>  18
<212>  DNA
<213>  Homo sapiens

<400>  5
gtgacgcgca tgaatgga                                                      18


<210>  6
<211>  18
<212>  DNA
<213>  Homo sapiens

<400>  6
cccttggctg tggtttcg                                                      18

2

1

## Description

The present invention relates to an *in vitro* analysis method and diagnostic kit for the quantitative determination of the expression of KCNM genes or their transcription or expression products. In particular, the KCNMA1 and KCNMB1 genes that code for the alpha and beta-1 subunits of the maxi-K ion channel involved in human arterial hypertension.

### STATE OF THE PREVIOUS ART

Arterial hypertension is the chronic form of raised blood pressure. It is a disease which has no symptoms for a long time and if left untreated, it is possible that the first symptom shown could be a serious complication such as a myocardial infarction (one of the main risks), clots or arterial ruptures (which may lead to haemorrhages), damage to nerve cells, loss of memory, paralysis, etc. The kidney also suffers from the consequences of arterial hypertension and renal failure is more likely to occur in hypertensive patients than in normotensive ones. The small vessels of the eye fundus (which are seen with an ophthalmoscope), can also be threatened by hypertension, their rupture causes haemorrhages, even leading to loss of sight. All these risks may be avoided if the hypertension is suitably treated and controlled.

The association existing between blood pressure and obstructive sleep apnea, hypoxia and respiratory disorders related to sleep, has been demonstrated experimentally (cf. Davies, C.W.H. et al., Thorax 2000, vol. 55, pp. 736-740; Fletcher, E.C. J. Appl. Physiol 2001, vol. 90, pp. 1600-1605; Grote et al., J. Hypertens 2000, vol. 18, pp. 679-685). These types of illnesses are an information focus for cardiovascular and arterial hypertension problems.

Ion channels are one of the advances on the structure and function of proteins that have arisen from molecular genetic research. These channels belong to a family of proteins which form macromolecular tunnels through the plasma membrane and are responsible for controlling the flow of ions to the exterior or interior of cells.

Previous observations that associated the beta-1 subunit of the maxi-K ion channel (expressed by the KCNMB1 gene) with hypertension are based on qualitative changes, such as the non-existence of the gene in mice, with a hypertensive state appearing in knockout mice for the beta-1 subunit, or such as the change in a single amino acid caused by a point mutation in humans (protection against diastolic hypertension in carriers of that mutation) (cf. Fernández-Fernández, J.M. et al., J.Clin.Invest 2004, vol. 113, pp. 1032-1039).

Currently, to carry out a study of the possible changes in the levels of expression of genes of vascular interest, it is done aggressively, by means of a biopsy to obtain tissue from the arterial wall.

### DESCRIPTION OF THE INVENTION

The invention deals with, in general, the problem of finding a quantitative method for the determination and/or detection of human arterial hypertension, in cells whose extraction process may not be aggressive for the patient.

The solution provided is based on the fact that the authors of the present invention have discovered, surprisingly, that the expression of KCNM genes, in particular, the KCNMA1 and KCNMB1 genes or their products of transcription or expression (RNA or protein) in peripheral blood leukocytes (PBL), can be quantified for the detection of human arterial hypertension.

KCNMA1 and KCNMB1 are two genes that code for two proteins which, upon being assembled, form the maxi-K ion channel.

Among the possible applications of the present invention it is included providing a diagnostic kit for human arterial hypertension in PBL, along with the use of PBL for the quantification of the genes that code for ion channels of importance in the vascular wall and not strictly speaking of the leukocyte, enabling the PBL to be used as remote controls of the regulation of these genes, which code for vascular ion channels.

In accordance with one aspect of the present invention, it is provided a method for the diagnosis of human arterial hypertension by quantitatively detecting the levels of expression of the KCNM genes in peripheral blood leukocytes (PBL) from a sample of whole blood. The PBL are obtained using any traditional method, well known in the field, such as a blood extraction.

The method provided by this invention, comprises the quantitative detection of the expression levels of the KCNM genes, in particular the KCNMA1 and KCNMB1 genes. Where these genes code for proteins for the alpha and beta 1 units of the maxi-K ion channel.

The term "maxi-K ion channel", as and when used in this description must be understood, in general, as a channel composed of a pore (a protein called alpha subunit) and a regulatory subunit (a protein called beta1 subunit) encoded respectively by the KCNMA1 and KCNMB1 genes, which allows the potassium ion to pass through the plasma membrane of the cell.

"Leukocytes in peripheral blood," as and when used in this description must be understood, in general, as white blood cells that circulate in the bloodstream, and that are mainly mononuclear of the lymphocyte type.

The expression of the KCNMA1 and KCNMB1 genes can be evaluated by any appropriate conventional method, for example, by determining the mRNA level corresponding to the KCNMA1 and KCNMB1 genes (mRNA of KCNMA1 and mRNA of KCNMB1).

In the sense used in this description, "to determine the mRNA level of KCNMA1 and the mRNA level of KCNMB1" includes any method that enables the level of mRNA to be measured or estimated which can be expressed as the KCNMA1 protein and the KCNMB1 protein in PBL cells. In this case, the level of mRNA of KCNMA1 and the mRNA of KCNMB1 will provide information relating to the prognosis of vascular diseases such as arterial hypertension.

The determination of the level of mRNA of KCNMA1 and mRNA of KCNMB1 can be determined by using any appropriate conventional method, for example, Northern blot analysis, S1 nuclease mapping, polymerase chain reaction (PCR), reverse transcription-polymerase chain reaction (RT-PCR), reverse transcription ligase chain reaction (RT-LCR), hybridization or microarrays.

In one embodiment of the method, the expression of the mRNA of KCNMA1 and the mRNA of KCNMB1 is assessed using reverse transcription-polymerase chain reaction (RT-PCR). In a preferred embodiment, this determination is carried out using PCR primers which amplify a DNA fragment of the KCNMA1 gene selected from the group made up of the sequences SEQ ID NO: 1 and SEQ ID NO: 2.

Another embodiment of the diagnostic method comprises a pair of PCR primers which amplify a DNA fragment of the KCNMB1 gene, selected from the group made up of the sequences SEQ ID NO: 3 and SEQ ID NO: 4.

Another embodiment of the diagnostic method comprises a pair of PCR primers which amplify a DNA fragment of the regulator region of the 28S gene of ribosomal RNA, selected from the group made up of the sequences SEQ ID NO: 5 and SEQ ID NO: 6

The ribosomal mRNA of the 28S subunit is considered a "housekeeping" gene. And this term "housekeeping gene" in this description means a gene whose expression level (in this case the level of its mRNA) does not vary significantly in the same person despite being subjected to different experimental conditions.

The invention also provides a method to evaluate the expression of the KCNMA1 and KCNMB1 genes by determining the expression level of the proteins corresponding to the aforementioned genes in PBL. Where the expression of the proteins corresponding to the KCNMA1 and KCNMB1 genes is carried out by any appropriate method, for example, using techniques based on antibodies, techniques based on *in vivo* diagnostic imaging, flow cytometry, proteomics, etc.

In a preferred embodiment of the method, the evaluation of the protein expression is carried out using flow cytometry with specific antibodies.

One aspect of the present invention comprises a pair of PCR primers which amplify a DNA fragment in the coding region of the KCNMA1 gene, selected from the group made up of the sequences SEQ ID NO: 1 and SEQ ID NO: 2.

Another aspect of the present invention comprises a pair of PCR primers which amplify a DNA fragment in the coding region of the KCNMB1 gene, selected from the group made up of the sequences SEQ ID NO: 3 and SEQ ID NO: 4.

Another aspect of the present invention comprises a pair of PCR primers which amplify a DNA fragment of the regulator region of the 28S gene of ribosomal RNA, selected from the group made up of the sequences SEQ ID NO: 5 and SEQ ID NO: 6

Yet another aspect of the present invention comprises a kit for the diagnosis of human arterial hypertension in a whole blood sample, which comprises primer oligonucleotides made up of the sequences SEQ ID NO: 1, SEQ ID NO: 2, SEQ ID NO: 3, SEQ ID NO: 4, SEQ ID NO: 5, SEQ ID NO: 6.

Yet another aspect of the present invention comprises the use of human peripheral blood leukocytes to evaluate the effect of substances with potential modulator ability over the expression of the genes that code for ion channels of cardiovascular interest. The gene types evaluated in the leukocyte are known ion channels of interest in the vascular area and not exactly of the leukocyte. Thus, for example, changes in molecules that circulate in the blood (as a decrease in arterial oxygen can be) cause a decrease in a gene, as KCNMB1 can be, in the cells of the vascular smooth muscle layer extractable by biopsy, which is identified by the effect that it causes on this same gene in cells that can be extracted and analyzed as easily as are PBL.

A preferred embodiment of the present invention is the use of PBL to quantify gene expression of cardiovascular interest detected in vascular smooth muscle.

Another preferred embodiment of the present invention is the use of PBL to quantify gene expression (like for example KCNM genes), using mRNA and/or proteins, as prognostic markers

Yet another embodiment of the present invention is the use of PBL to quantify gene expression (like for example KCNM genes), using mRNA and/or proteins, as therapeutic targets.

Throughout the whole of the description and the claims of the specification, the word "comprises" and variations of the same, are not expected to exclude other aspects of the present invention, which would be obvious to an expert in the field upon looking at the description.

The detailed presentation of the embodiments and the figures that follow are provided as a means of illustration and are not intended to be limiting factors of the present invention.

### DESCRIPTION OF THE FIGURES

Fig. 1 shows the regression lines of the amplification of the KCNM (KCNMA1 and KCNMB1) and 28S genes, from which the quantity of expression in each gene in the sample is calculated. It is constructed with relative amounts of complementary DNA, by dilutions of 1/2, 1/4, 1/8 and 1/16 (v/v) for the KCNM genes and dilutions of 1/20, 1/40, 1/80 and 1/160 for the 28S gene. t.c. is the threshold cycle, that is, the cycle of the quantitative PCR from which it starts to detect the gene; and C_{DNA} is the amount of complementary DNA that serves as a template in the quantitative PCR reaction.
Fig. 2 shows the amplification band of the KCNMB1 gene, marked as β1, once amplified by quantitative PCR in human mammary artery (AM), in human leukocytes (PBL) and human lymphocytes from a tumor line clone (Jurkat).
Fig. 3 shows the level of expression, measured by quantitative PCR, of the KCNMB1 gene in the indicated cell types (in PBL and Jurkat) after incubation for 24 hours in hypoxia of 1%. At 100% expression level of each type cell it has a normoxia of 21 % oxygen. The PBL are peripheral blood leukocytes and Jurkat is a cell line established by tumoral human lymphocytes.
Fig. 4 shows the level of expression, measured by quantitative PCR, of the KCNMB1 gene in the indicated cell types after incubation for 24 hours in hypoxia of 1%. At 100% expression level of each type cell it has a normoxia of 21 % oxygen. M are arterial myocytes, AOR-A7r5 is a cell line established in the aorta of the A7r5 rat, AOR-SMC is the first culture from the rat's aorta, ABR is the first culture from the rat's basilar artery and AMH is the first culture from human mammary artery.
Fig. 5 shows the bivariate correlations between the mean systolic blood pressure (P) and the minimal arterial oxygen saturation (Sm O₂), measured in patients with OSAS.
Fig. 6 shows the bivariate correlations between the messenger RNA level (β1 mRNA) of the KCNMB1 gene and the minimal arterial oxygen saturation (Sm O₂), measured in patients with OSAS.
Fig. 7 shows the bivariate correlations between the messenger RNA level (β1 mRNA) of the KCNMB1 gene and the mean systolic blood pressure (P), measured in patients with OSAS.

### DETAILED EXPLANATION OF EMBODIMENTS

The invention will be illustrated below using the following assays performed by the inventors, which demonstrate the specificity and efficacy of the method and diagnostic kit for human arterial hypertension.

### EXAMPLE 1 - Quantitative detection of the mRNA of the KCNMA1 and KCNMB1 genes in PBL.

The present example was carried out on patients with obstructive sleep apnea syndrome (OSAS), also known as sleep apnea/hypopnea syndrome (SAHS).

After obtaining informed consent, 1 ml of whole blood was extracted by peripheral venepuncture from each subject in the study.

The mRNA of the human leukocytes was obtained in the first hour after the blood extraction using OIAamp^{®} RNA Blood Mini kit (Qiagen) commercial kit and 0.8 ug of complete RNA was used for the reverse transcription (RT) reaction in a final volume of 60 ul, using Superscript^{™} First-Strand Synthesis System for RT-PCR (Invitrogen), following the manufacturer's instructions.

The quantitative PCR was performed with SYBR^{®} Green PCR Master mix (Applied Biosystems) in a reaction mixture of 30 ul containing: 1 x SYBR^{®} Green PCR Master mix (which includes ROX as a passive reference fluorochrome), primer oligonucleotides selected from Table 1 (150-300 nM), and 2 ul of a 1/8 (v/v) dilution (or 1/80 (v/v) dilution in the case of 28S amplification) of the final volume of the RT.

**Table 1 - Primer oligonucleotides for amplification by PCR.**

| Type | Access No. | SEQ ID NO: | Primers | Position |
|---|---|---|---|---|
| αMaxiK | NM_002247 | 1 | TCAGCAGATGCATGCCTGAT | 1624-1643 |
| αMaxiK | NM_002247 | 2 | TCTCATGATATTCGAGGCATCCT | 1679-1701 |
| | | | | |
| β1MaxiK | NM_004137 | 3 | GTGTGCCGTCATCACCTACTACAT | 475-498 |
| β1MaxiK | NM_004137 | 4 | CCTGGGTCCACACGCTTT | 531-548 |
| | | | | |
| 28S rRNA | M11167 | 5 | GTGACGCGCATGAATGGA | 3733-3790 |
| 28SrRNA | M11167 | 6 | CCCTTGGCTGTGGTTTCG | 3827-3844 |

The real time PCR were carried out in an Abiprism 7000 (Applied Biosystems) apparatus using the following program:
- 10 min at 95° C, and then 40 cycles of 15 seconds at 95° C and 1 minute at 60° C.

The real time PCR cycle from which the fluorescent signal starts (threshold cycle) correlates with the initial quantity of mRNA of the sample. Thus there was a higher gene expression the earlier the threshold cycle. Standardization of the values was carried out simultaneously in parallel with a gene considered as "housekeeping" as is the 28S subunit of ribosomal RNA.

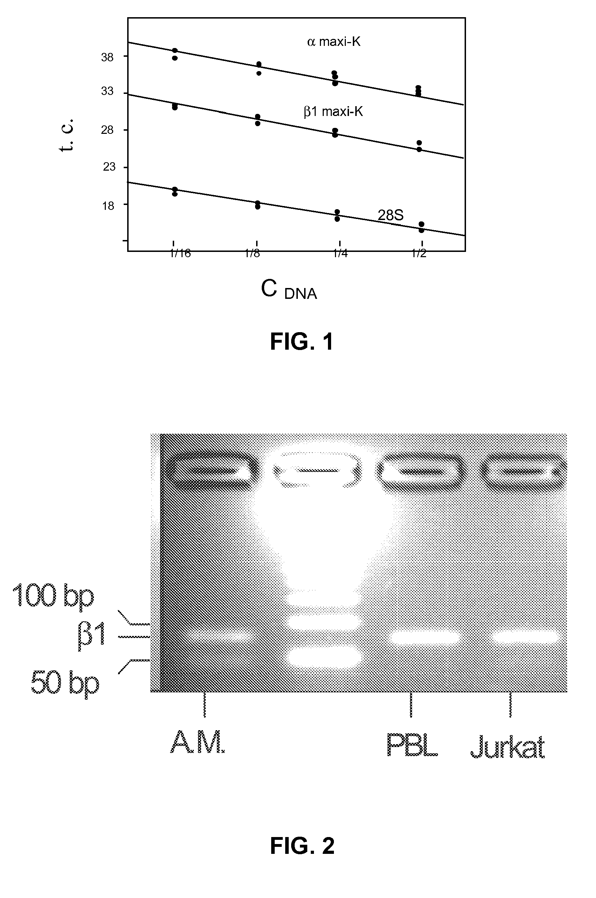

The products amplified by this PCR were analyzed using the software of the Abiprism 7000 apparatus itself. The analysis of the dissociation curves for each gene showed a single peak with the expected dissociation temperature in all the samples. Quantification was carried out by means of interpolation, using a standard regression line of the threshold cycle values obtained from the serial dilutions 1/4, 1/8 and 1/16 (v/v) for the KCNMA1 and KCNMB1 genes (or 1/40, 1/80 and 1/160 (v/v) in the case of the 28S housekeeping gene) from the final volume of the RT (Fig. 1).

### EXAMPLE 2 - Design of the diagnostic kit for human arterial hypertension.

The oligonucleotide primer pairs, according to Table 1, are included in the diagnostic kit, for the amplification of each gene, KCNMA1 (αMaxi-K), KCNMB1 (β1 Maxi-K) and 28S of ribosomal RNA, using real time quantitative PCR, and they were designed with the program Primer Express 2.0 of Applied Biosystems.

### EXAMPLE 3 - Regulation of the beta 1 subunit expression by O₂ tension in human leukocytes of peripheral blood and beta1 testing in patients with SAHS.

The correlation between the level of expression of the maxi-K channel beta 1 subunit and the severity of the disease was investigated in patients with SAHS, after obtaining informed consent. PBL were used to determine the expression and regulation by hypoxia of the maxi-K channel beta 1 subunit, given that performing a biopsy to obtain arterial smooth muscle is an aggressive technique and is not risk free.

The peripheral blood leukocytes were obtained without any complications from the peripheral blood of the subjects studied, these PBL are in direct contact with the O₂ tension and contain potassium currents attributable to maxi-K channels (cf. Brink, P.R., et al. Plugers Arch. Eur. J. Physiol 1990, vol. 417, pp. 349-351; Ahluwalia, J et al., Nature 2004, vol. 427, pp. 853-856).

The mRNA of the beta1 subunit was amplified in both the PBL and the lymphocytes from the cloned line called Jurkat with a suitable bandwidth identified by RT-PCR (Fig. 2), which was later confirmed by sequencing the PCR fragments.

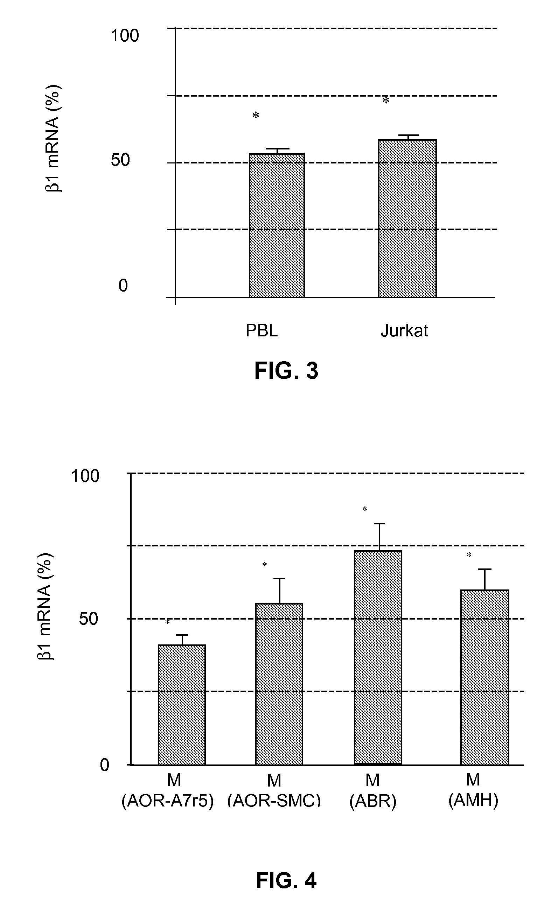

The presence of the beta1 protein is confirmed by immunocytochemistry using a specific antibody against the beta1 subunit. The exposure to hypoxia of the two types of human leukocytes led to a 50% decrease in the expression of mRNA of the beta1 subunit (Fig. 3), a similar effect to that observed in arterial smooth muscle cells (Fig. 4).

Based on these results, the beta1 mRNA levels in the PBL of 10 patients with untreated SAHS was systematically determined. The patients were males, with a mean age of 45.2±2.9 years and with a body mass index of 32.8±1.3 kg/m². The apnea-hypopnea and desaturation indices were 64.8±6.3 and 65±6.3, respectively.

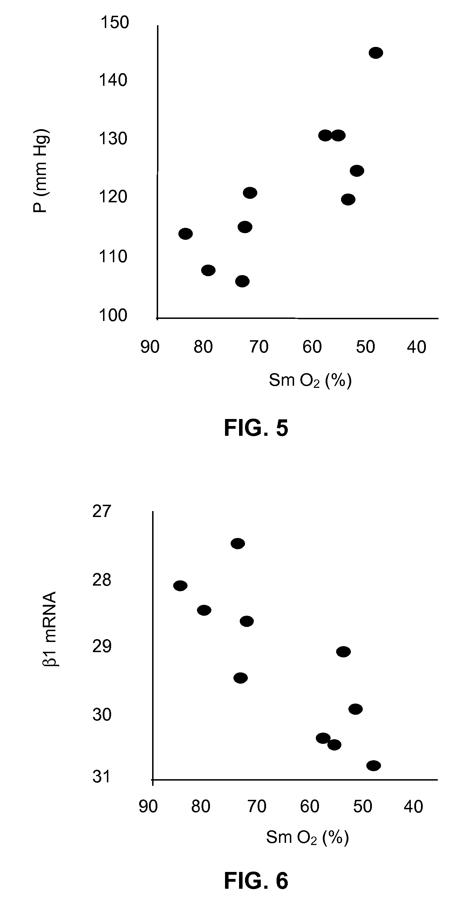

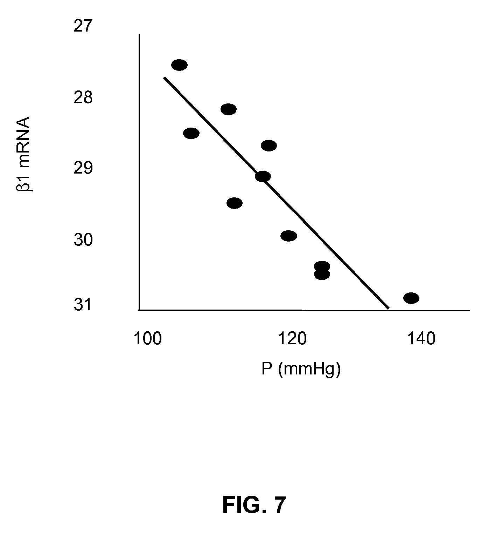

The mean values of the rest of the variables measured during the individual polysomnography study and the study of the blood pressure recorded for 24 hours are shown in Table 2. The mean systolic blood pressure (Fig. 5) and the PBL beta1 mRNA levels (Fig. 6, r=0.64, p<0.05) correlated, although inversely, with the minimum nocturnal arterial O₂ saturation (Sm O₂). A strong correlation (r=0.80; p<0.01) was detected between the beta1 mRNA levels and the systolic blood pressure (Fig. 7).

These data suggest that chronic intermittent hypoxia leads to a decrease in the expression of the maxi-K channel beta1 subunit and this phenomenon may lead to arterial hypertension.

**Table 2. Parameters measured in patients with OSAS during the polysomnographic study and the continuous 24 hour monitoring of the blood pressure (mean ± standard error of the mean, n=10).**

| Sleep study | | Blood pressure monitoring | |
|---|---|---|---|
| Baseline SatO₂ | 92.8 ± 0.8 | Systolic blood pressure | 124 ± 3.4 |
| Min. SatO₂ | 65.9 ± 4.3 | Diastolic blood pressure | 79.1 ± 1.5 |
| Desaturation index | 65.0 ± 6.3 | Heart rate | 78.3 ± 3.5 |
| CT90 | 22.8 ± 7.4 | % systolic readings >135 | 21.6 ± 7.8 |
| Arousal index | 46.7 ± 4.7 | % diastolic readings >85 | 31.4 ± 5.9 |
| Apnea-Hypopnea Index | 64.8 ± 6.3 | | |

The invention not only demonstrates that a molecular parameter (level of beta1 expression) correlates with an etiopathogenic factor (as is hypoxia) and with an extremely important biological variable (blood pressure), but also that it implies that quantitative changes in the level of beta1 expression could determine human blood pressure values.

To establish that a quantitative difference in the beta1 subunit of the maxi-K channel in humans correlates with a different blood pressure demonstrates not only the value as a potential diagnostic marker, but also the high therapeutic interest there could be in drugs that modulate the increase in expression of that gene or which could directly activate the encoded protein.

## Claims

1. A diagnostic method for human arterial hypertension, **characterized in that** it quantitatively detects the expression levels of the KCNM genes in peripheral blood leukocytes in a sample of whole blood.

2. Method according to claim 1, wherein the KCNM genes, in particular the KCNMA1 and KCNMB1 genes, code for proteins for the alpha and beta units of the maxi-K ion channel.

3. Method according to any of the claims 1 and 2, wherein the evaluation of the expression of the KCNMA1 and KCNMB1 genes is carried out by determining the level of mRNA corresponding to the KCNMA1 and KCNMB1 genes (KCNMA1 mRNA and KCNMB1 mRNA).

4. Method according to claim 3, wherein the determination of the KCNMA1 mRNA and KCNMB1 mRNA is carried out using Northern blot analysis, mapping with S1 nuclease, polymerase chain reaction (PCR), reverse transcription in combination with polymerase chain reaction (RT-PCR), reverse transcription in combination with ligase chain reaction (RT-LCR), hybridization or microarrays.

5. Method according to claim 4, wherein the determination of the KCNMA1 mRNA and KCNMB1 mRNA is carried out by reverse transcription in combination with polymerase chain reaction (RT-PCR).

6. Method according to any of the claims 3 to 5, wherein PCR primers that amplify a DNA fragment of the KCNMA1 gene are used, selected from the group made up of the sequences SEQ ID NO: 1 and SEQ ID NO: 2.

7. Method according to any of the claims 3 to 5, wherein PCR primers that amplify a DNA fragment of the KCNMB1 gene are used, selected from the group made up of the sequences SEQ ID NO: 3 and SEQ ID NO: 4.

8. Method according to any of the claims 3 to 5, wherein PCR primers that amplify a DNA fragment of the 28S gene of ribosomal RNA are used, selected from the group made up of the sequences SEQ ID NO: 5 and SEQ ID NO: 6.

9. Method according to any of the claims 1 and 2, wherein the evaluation of the expression of the KCNMA1 and KCNMB1 genes is carried out by determining the level of expression of the proteins corresponding to these genes.

10. Method according to claim 9, where the evaluation of the expression of the proteins is carried out by using techniques based on the use of antibodies, techniques based on *in vivo* imaging diagnostics, flow cytometry or proteomics.

11. Method according to claim 10, wherein the evaluation of the expression of the proteins is carried out by using flow cytometry with specific antibodies.

12. A pair of PCR primers that amplify a DNA fragment in the coding region of the KCNMA1 gene, selected from the group made up of the sequences SEQ ID NO: 1 and SEQ ID NO: 2.

13. A pair of PCR primers that amplify a DNA fragment in the coding region of the KCNMB1 gene, selected from the group made up of the sequences SEQ ID NO: 3 and SEQ ID NO: 4.

14. A pair of PCR primers that amplify a DNA fragment in the coding region of the 28S gene of ribosomal RNA, selected from the group made up of the sequences SEQ ID NO: 5 and SEQ ID NO: 6.

15. A quantitative diagnostic kit for human arterial hypertension, **characterized in that** it comprises oligonucleotide primers according to claims 12, 13 and 14.

16. Use of peripheral blood leukocytes **characterized in that** it evaluates the effect of substances with potential modulator ability over the expression of the genes that code for ion channels of cardiovascular interest.

17. Use of leukocytes, according to claim 16, to quantify the expression of genes of cardiovascular interest detected in vascular smooth muscle.

18. Use of leukocytes, according to claim 16, to quantify the expression of genes, using mRNA and/or proteins, as prognostic markers.

19. Use of leukocytes, according to claim 16, to quantify the expression of genes, using mRNA and/or proteins, as therapeutic targets.
